# EUROPEAN PATENT SPECIFICATION

(11) **EP 1 501 530 B1**
(45) Date of publication and mention of the grant of the patent: **18.02.2009**
(21) Application number: 03718033.8
(22) Date of filing: 21.03.2003
(51) Int. Cl.: A61K 38/00, A61P 27/02

(54) **PROSTAGLANDIN F2 ALPHA ANALOGS IN COMBINATION WITH ANTIMICROBIAL FOR TREATING GLAUCOMA**
PROSTAGLANDIN F2 ALPHA ANALOGA IN KOMBINATION MIT EINEM ANTIMIKROBIELLEN MITTEL ZUR BEHANDLUNG VON GLAUKOM
ANALOGUES DE LA PROSTAGLANDINE F2 ALPHA EN COMBINAISON AVEC DES ANTIMICROBIENS POUR LE TRAITEMENT DU GLAUCOME

(30) Priority: 21.03.2002 US 367071 P
(43) Date of publication of application: 02.02.2005
(73) Proprietor: Cayman Chemical Company, Ann Arbor, MI 48108 (US)
(72) Inventor: MAXEY, Kirk, M., Fort Collins, CO 80526 (US); JOHNSON, Jennifer, Ann Arbor, MI 48108 (US)
(74) Representative: Jacob, Reuben Ellis
(86) International application number: PCT/US2003/008935
(87) International publication number: WO 2003/079997

(56) References cited:
- EP-A- 0 563 844
- WO-A-02/00839
- WO-A-02/38158
- US-A- 5 753 620
- US-A- 5 889 052
- US-A1- 2002 035 149
- US-B1- 6 329 426

## Description

The present invention relates to novel compositions of matter, and more particularly, to the novel combination of prostaglandin compounds of the F-series (PGF) with antimicrobial peptides, and the use of such compositions for the treatment of increased intraocular pressure, such as that caused by glaucoma and the reduction of ocular hypertension.

The prostaglandins are a family of 20 carbon atom fatty acids, being structural derivatives of prostanoic acid, which exhibit useful activity in a wide variety of biological systems. Accordingly, prostaglandins represent useful pharmacological agents in the treatment and prevention of a wide variety of disease conditions. For a fuller discussion of prostaglandins and their uses, see Oates, et al., New England J. Med. Vol. 319, No. 11, pages 689-698 and Vol. 319, No. 12, pages 761-768 (1988) and references cited therein.

The prostaglandins are a family of 20 carbon atom fatty acids, being structural derivatives of prostanoic acid, which exhibit useful activity in a wide variety of biological systems. Accordingly, prostaglandins represent useful pharmacological agents in the treatment and prevention of a wide variety of disease conditions. For a fuller discussion of prostaglandins and their uses, see Oates, et al., New England J. Med., Vol. 319, No. 11, pp. 689-698 and Vol. 319, No. 12, pp. 761-768 (1988) and the references cited therein.

Prostaglandin F_{2α} (PGF_{2α}) is a naturally-occurring prostaglandin which is widely manufactured and sold under a variety of trade names as an abortifacient, among other uses. See monograph 8065, page 1354 of The Merck Index, 12th edition (1996). It is also well known in the art that naturally-occurring prostaglandins can be topically applied to lower intraocular pressure. However, naturally-occurring prostaglandins generally cause inflammation and surface irritation of the eye.

Analogs of PGF_{2α} which incorporate aromatic groups and other substituents into the omega chain have been prepared and found to be more potent and selective pharmacologic agents than the naturally-occurring compounds. An exemplary PGF_{2α} analog of this type is fluprostenol, 16-(meta-trifluoromethylphenoxy)-17,18,19,20 tetranor PGF_{2α}, and its pharmacologic profile is described in monograph 4231, page 711 of The Merck Index, 12th edition (1996). Unfortunately, the known synthetic analogs also produce the same undesirable side effects as the naturally-occurring compounds. There is, therefore, a need in the art for less inflammatory compounds for reducing intraocular pressure.

A number of simple PGF_{2α} analog esters have been found to be potent and selective agents useful for the treatment of ocular hypertension. For example, latanoprost is the isopropyl ester of 13,14-dihydro-17-phenyl-18,19,20-trinor PGF_{2α} and is widely marketed for the clinical treatment of glaucoma under the trade name Xalatan. See monograph 5387, page 918 of The Merck Index, 12th edition (1996). Likewise, the isopropyl ester of fluprostenol and of similar PGF_{2α} analogs, such as cloprostenol, are specifically claimed as ocular antihypertensive agents in U.S. patent 5,665,773. The structures of naturally-occurring PGF_{2α} (Structure I), fluprostenol (Structure II), and latanoprost (Structure III), and bimatoprost (Structure IV) are shown hereinbelow. For a review of these agents, see Lindén and Alm, Drugs and Aging, Vol.14, No. 5, pp. 387-398 (1999).

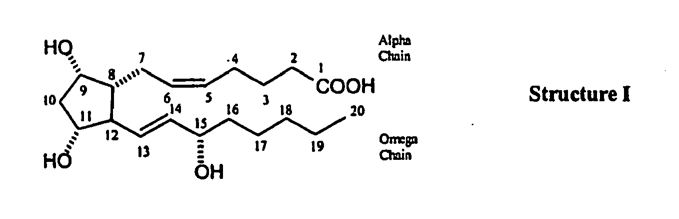

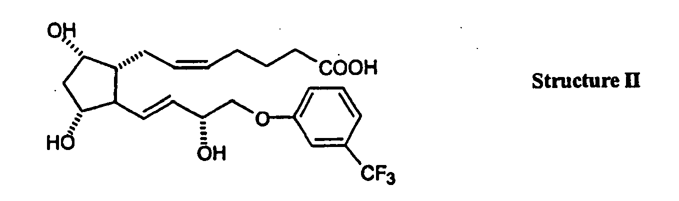

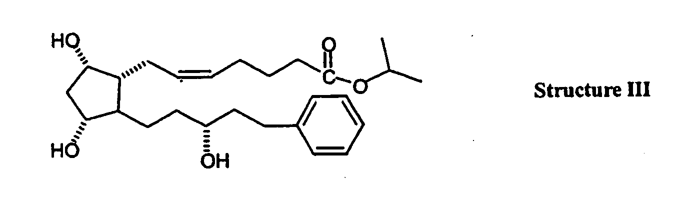

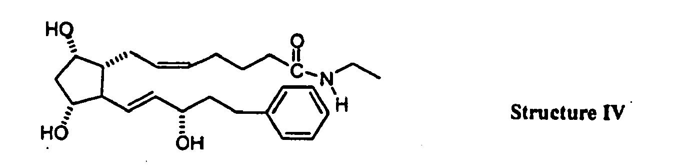

The amides and esters of PGF_{2α} and its analogs are believed to act as prodrugs in the eye, in that the ester form which is administered is hydrolyzed by endogenous ocular esterase enzymes, releasing the PGF_{2α} analog free acid as the active pharmacologic agent. However, this also releases a toxic and irritant small aliphatic alcohol such as isopropanol or methanol into the eye. In the case of bimatoprost, ethylamine is released into the eye. While highly effective in reducing intraocular pressure, all of the drugs currently in use cause a significant level of eye irritation.

In addition to the foregoing, the isopropyl esters of PGF_{2α} analog compounds such as latanoprost and fluprostenol are highly viscous, glassy oils which are difficult to handle and to formulate into ophthalmic solutions. Worse yet, these compounds are prone to the retention of potentially toxic process solvents. There is, therefore, a need for a form of the prostaglandin analogs which is easier to handle and which does not release an irritant alcohol upon hydrolysis.

In addition to the irritation caused by the prostaglandins themselves, and particularly the naturally-occurring and synthetic prostaglandins of the type presently on the market, the preservatives typically used in ophthalmic solutions are known to irritate a large percentage of the population. Thus, despite the fact that the prostaglandins represent an important class of potent therapeutic agents for the treatment of glaucoma, the unwanted side effects of these drugs, particularly ocular irritation and inflammation, are the chief reasons for patient withdrawal from the use of these drugs. There is, therefore, a need for a less irritating ophthalmic formulation for the therapeutic prostaglandin compounds.

This problem has been addressed in the art by creating novel prostaglandins that, in their free acid active form, are more selective and less prone to bind to, and activate, the receptors responsible for side effects. As an example, internal esters of PGF_{2α} analogs have been devised by creating a carbon-oxygen bond between the alcohol group at C-15 and C-1 carboxylic acid. This creates a macrocyclic lactone that has novel and desirable characteristics. In fact, some of the novel analogs form highly crystalline structures that are advantageously easy to formulate into ophthalmic solutions. The hydrolysis of these PGF_{2α} analog 1,15-lactones releases only the active PGF_{2α} analog free acid, without the production of a small aliphatic alcohol coproduct. Thus, these compounds are ideal and unique prodrugs for the treatment of glaucoma and other disorders causing an increase in intraocular pressure in the eyes of humans or animals.

Examples of these newly developed macrocyclic internal 1,15-lactones of fluprostenol and related PGF analogs are shown hereinbelow as Structures V-VII. For a more complete description, however, see International Patent Publication No.0 1/57015 published under the Patent Cooperation Treaty on August 9, 2001.

Although the macrocyclic internal 1,15-lactones of fluprostenol and related PGF analogs themselves are less irritating, the synthetic preservatives commonly used in ophthalmic solutions, such as benzalkonium chloride and thimerosal, are irritating to many patients, especially those with allergy or chemical hypersensitity. There is, therefore, a need for ophthalmic solutions that do not contain synthetic chemical preservatives.

Antimicrobial peptides, such as lactoferrin and lactoferricin, are produced naturally in the human eye. However, these antimicrobial peptides have not been used heretofore as a preservatives in ophthalmic solutions.

Antimicrobial peptides have been known to science since the early 1980s, when they were discovered in insects (see, Steiner, et al., Nature, Vol. 292, pages 246-268 (1981); in amphibians (see, Simmaco, et al., Biopolymers, Vol. 47, pages 345-450 (1998) and Zasloff, M. Proc. Natl. Acad. Sci., USA 84, pages 5449-5453 (1987); and in humans (see, Selstad, et a/., J. Biol. Chem., Vol. 267, pages 4292-4295 (1992)). Perhaps the best known antimicrobial peptide is lactoferrin, found naturally in human tears and in human milk, and the defensive antimicrobial peptide lactoferricin, which is derived from it. A listing of the names and structures of all of the currently known antimicrobial peptides, can be found on the internet at the following the web site: http://www.bbcm.univ,trieste.it/~tossi/antimic.html.

Antimicrobial peptides have applications as antibiotics, for example, Pexiganan, a derivative of the defensive antimicrobial peptide from the skin of the African clawed frog, is under development as a topical antibiotic for treating diabetic skin ulcers. However, the combination of antimicrobial peptides with prostaglandin ester, amide, and especially internal lactone prodrugs, in ophthalmic formulations, is novel and results in a synergistic and beneficial reduction in the incidence of irritant side effects. These irritant side effects include, melanocyte hyperplasia, which is of concern in that it could lead to the more dangerous cellular dysplasia, with chronic application of the drug. This irritant side effect has also been recognized more recently in the form of iridial melanocyte hyperplasia and hyperpigmentation which has been documented in patients treated with ophthalmic solutions of isopropyl esters, such as Unoprostone (See page 2 of NDA 21-214 for Rescula^{™} Ciba Vision). Thus, the benefits of this invention extend beyond improved patient comfort, compliance, and acceptance, but also to patient safety.

It is, therefore, an object of this invention to provide improved ophthalmic formulations, and particularly ophthalmic formulations for prostaglandin compounds that are useful for the reduction of intraocular pressure with less inflammation and toxic side effects.

### SUMMARY OF THE INVENTION

The foregoing and other objects are achieved by this invention which provides, in a composition of matter aspect, a combination of prostaglandin compounds and antimicrobial peptides, and more particularly a combination of prostaglandin compounds of the F-series (PGF) with antimicrobial peptides. In order to improve on the therapeutic index of prostaglandin-containing ophthalmic formulations, and in particular, to decrease the incidence of unwanted side effects, such as hyperemia, irritation, and inflammation of the conjunctiva, the active prostaglandin ingredients have been formulated into ophthalmic solutions which contain no synthetic chemical preservatives, such as benzalkonium chloride, BHT, or similar irritating phenyl-aromatic preservatives, but which instead contain antimicrobial peptides, and in particularly preferred embodiments, antimicrobial peptides of human derivation, such as lactoferrin. The formulation is, therefore, ideally suited to be dispensed in multidose format.

The formulation which is, in preferred embodiments, a combination of a prostaglandin prodrug with an antimicrobial peptide preservative, further reduces the incidence of irritant side effects, results in better patient compliance, and reduced ocular inflammation. The term prodrug is meant to include a prostaglandin ester, prostaglandin amide, or most preferably, a prostaglandin lactone, In particularly preferred embodiments, the prostaglandin compound is a macrocyclic internal 1,15-lactone, and in particular internal 1,15-lactones of PGF_{2α} analogs, such as the 16-aryloxy prostaglandin analogs, illustratively fluprostenol or cloprostenol.

For the purposes of this patent, however, the term "prostaglandin" is intended to mean any one of the prostanoic acid derivatives which include the ring type A, B, C, D, E, F, G, H, I, J and K, but most particularly those of the F-type. The term "derivative" is intended to mean all compounds which have a chemical affinity, resemblance, or structural character which clearly associates them with the prostanoids and in particular, prostanoic acid or PGF_{2α}. The term "analog" is intended to mean any somewhat modified version of a natural product, in this case a prostaglandin, or a related synthetic analog, wherein a number of atoms such as carbon, hydrogen, oxygen or heteroatoms such as nitrogen, sulfur or halide have been added or deleted from the parent structure, so as to yield a new molecular compound.

The preferred prostaglandin compounds for use in the formulation composition of the present invention have the general Formula I: 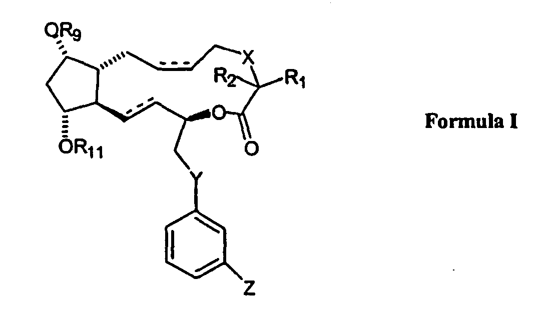 wherein X is O, S, NH or CH₂;
R₁ and R₂ are the same and are either H, CH₃ or F;
R₉ is H, or C₁-C₂₀ straight chain, saturated or unsaturated or branched acyl;
R₁₁ is H, or C₁-C₂₀ straight chain, saturated or unsaturated or branched acyl;
---- represents any combination of a single bond, or a *cis* or *trans* double bond;
Z is H, Cl, Br, I, CF₃, CH₃, or C₁-C₁₀ straight chain or branched alkyl;
Y is O, S, NH or CH₂.

In preferred embodiments of the present invention, the prostaglandin component of the formulation is an internal 1,15-lactone that is an analog of the PGF_{2α} analog fluprostenol. In particular, analogs of fluprostenol, having the structure of Formula I wherein R₉ and R₁₁ are H; Y is one of the polar substituents O, S, or NH; and Z is CH₃ are highly crystalline and therefore, are readily formulated into ophthalmic solutions for topical application.

In specific preferred embodiments of the present invention, the prostaglandin component is the 1-15-lactone of fluprostenol (Structure V), the 1,15-lactone of cloprostenol (Structure VI) and the 1,15-lactone of latanoprost (Structure VII).

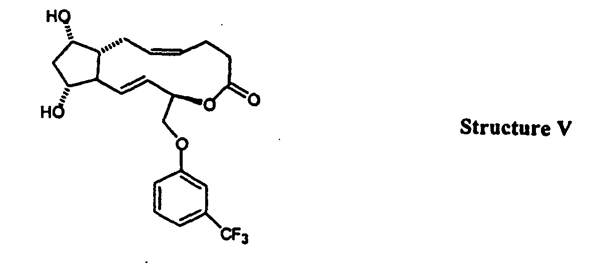

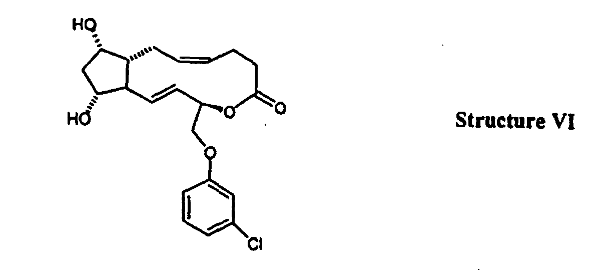

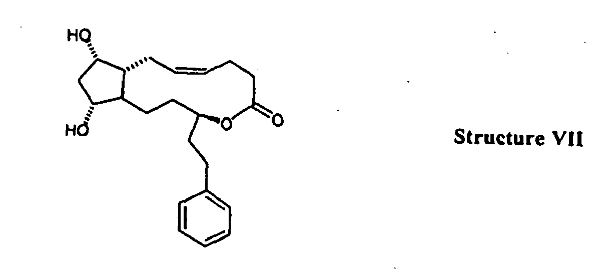

It is also contemplated that prostaglandin compounds for use in the formulation composition of the present invention have the general Formula II, and particularly include, the known prostaglandin analogs of Formula II: 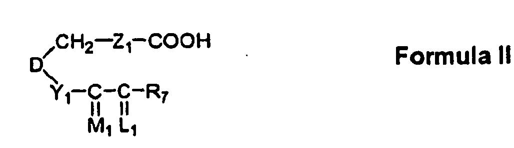 wherein D is 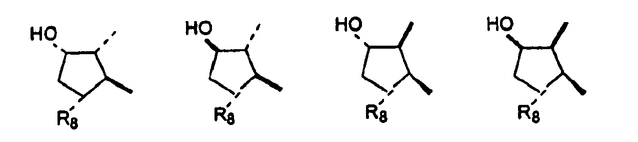 wherein R₈ is hydrogen or hydroxy;
wherein L₁ is 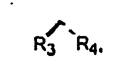 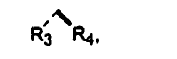 or a mixture of 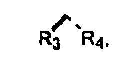 and 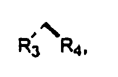 wherein R₃ and R₄ are hydrogen, methyl or fluoro, being the same or different, with the proviso that one of R₃ and R₄ is fluoro, only when the other is hydrogen or fluoro;
wherein M₁ is 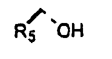 or 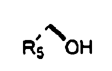 wherein R₅ is hydrogen or methyl;
wherein R7 is -(CH₂)*ₘ* -CH₃, wherein *m* is one to 5,
inclusive, cis-CH=CH-CH₂-CH₃, or 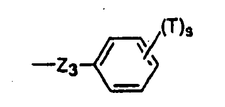 wherein T is chloro, fluoro, trifluoromethyl, alkyl of one to 3 carbon atoms, inclusive, or alkoxy of one to 3 carbon atoms, inclusive, the various T's being the same or different, *s* is zero, one, 2, or 3, and Z₃ is oxa or methylene, with the proviso, that not more than two T's are other than alkyl, and the further proviso that Z₃ is oxa only when R₃ and R₄ are hydrogen or methyl, being the same or different;
wherein Y₁ is trans -CH=CH-, -CH₂CH_{2-,}
cis-CH=CH-, or -C≡C-; and
wherein Z₁ is
(1) cis-CH=CH-CH₂-(CH₂)*_{g}*-CH₂-,
(20) cis-CH=CH-CH₂-(CH₂)*_{g}*-CF₂-,
(3) cis-CH₂-CH=CH-(CH₂)*_{g}*-CH₂-_{,}
(4) -(CH₂)₃-(CH₂)*_{g}*-CH₂-,
(5) -(CH₂)₃-(CH₂)*_{g}*-CF₂-,
(6) -CH₂-O-CH₂-(CH₂)*_{g}*-CH₂-, 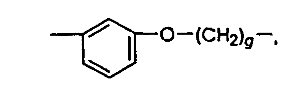
and 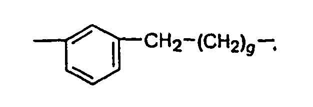 wherein *g* is one, 2, or 3.

It is to be understood that, irrespective of whether specifically designated herein, the most active stereoisomers of the prostaglandin compounds are preferred. Typically, the active isomers are known in the art to be those isomers that mimic naturally-occurring prostaglandins.

Any antimicrobial peptide, or protein, that is presently known, or that may be developed in the future, is within the contemplation of the invention for use as the preservative in improved ophthalmic formulations comprising prostaglandins of Formulae I and II. Illustrative antimicrobial peptides are listed in Table 1.

**Table I**

| **Representative Peptides** | |
|---|---|
| Cecropin A | KWKLFKKIEKVGQNIRDGIIKAGPAVAVVGQATQIAK-amide |
| Magainin 2 | GIGKFLHSAKKFGKAFVGEIMNS |
| Pexiganan | GIGKFLKKAKKFGKAFVKILKK-amide |
| Dermaseptin I | ALWKTMLKKLGTMALHAGKAALGAAADTISQGTQ |
| LL-37 | LLGDFFRKSKEKIGKEFKRIVQRIKDFLRNLVPRTES |
| Buforin II | TRSSRAGLQFPVGRVHRLLRK |
| Bactenecin 1 | RLCRIVVIRVCR |
| Thanatin | GSKKPVPIIYCNRRTGKCQRM |
| Brevinin IT | VNPIILGVLPKVCLITKKC |
| Ranalexin | FLGGLIKIVPAMICAVTKKC |
| Ranateurin 1 | SMLSVLKNLGKVGLGFVACKINKQC |
| Esculentin 1 | IFSKLGRKKIKNLLISGLKNVGKEVGMDWRTGIDIAGCKIKGEC |
| Tachyplesin | RWC₁FRVC₂YRGIC₂YRKC₁R-amide |
| Androctonin | RSVC₁RQIKIC₂RRRGGC₂YYKC₁TNRPY |
| Protegrin I | RGGRLC₁YC₂RRRFC₂VC₁VGR-amide |
| α-defensin (HNP3) | DC₁YC₂RIPAC₃IAGERRYGTC₂IYQGRLWAFC₃C₁ |
| β-defensin (TAP) | NPVSC₁VRNKGIC₂VPIRC₃PGSMKQIGTC₂VGRAVKC₁C₃RKK |
| θ-defensin | GFC₁RC₂LC₃RRGVC₃RC₂IC₁TR |
| Defensin (sapecinA) | ATC₁DLLSGTGINHSAC₂AAHC₃LLRGNRGGYC₂NGKAVC₃VC₁RN |
| Thionin (crambin) | TTC₁C₂PSIVARSNFNVC₃RIPGTPEAIC₃ATYTGC₂IIIPGATC₁PGDYAN |
| Defensin | QKLC1QRPSGTWSGVC2GNNNAC3KNQC4IRLEKARHGSC2NYVFPAHC3IC4 YFPC1 |
| Drosomycin | DC₁LSGRYKGPC₂AVWDNETC₃RRVC₄KEEGRSSGHC₂SPSLKC₃WC₄EGC₁ |
| Hepcidin | DTHFPIC₁IFC₂C₁GC₄C₁HRSKC₂GMC₃C₄KT |
| Bac5 | RFRPPIRRPPIRPPFYPPFRPPIRPPIFPPIRPPFRPPLGRPFRα |
| PR-39 | RRRPRPPYLPRPRPPPFFPPRLPPRIPPGFPPRFPPRFPα |
| Indolicidin | ILPWKWPWWPWRRα |
| Apidaecin | GNNRPVYIPQPRPPHPRI |
| Pyrrhocoricin | VDKGSYLPRPTPPRPIYNRN |
| Histatin 5 | DSHAKRHHGYKRKFHEKHHSHRGY |

In preferred embodiments, the antimicrobial peptides would be soluble in water, or at least easily formulated into dilute aqueous solutions. Particularly preferred antimicrobial peptides are those of human origin, and include lactoferrin and lactoferricin. Other useful antimicrobial peptides include Buforin II, pexiganan, Apidaecin, and Bactenecin, all of which are readily available and can be easily expressed.

Antimicrobial peptides are available commercially from sources such as Magainin Pharmaceuticals, Plymouth Reading, PA or Gem Pharmaceuticals, Birmingham, AL. Antimicrobial peptides can also be obtained by isolation from natural sources, by isolation as recombinant proteins from bacteria, or cultured mammalian or insect cells. Techniques for isolating or culturing antimicrobial peptides are available in the published literature and are known to a person of ordinary skill in the art.

The prostaglandin compounds of the present invention can be synthesized from commercially available starting materials as described in numerous publications known to those of skill in the art. Prostaglandins are also available from commercial sources, such as Cayman Chemical, Ann Arbor, MI or Chinoin, Budapest, Hungary. Useful prostaglandin compounds include those compounds specifically listed in the examples presented herein, such as 13,14-dihydro-17-phenyl-18,19,20-trinor FGF₂α isopropyl ester; fluprostenol 1,15-lactone; 13,14-dihydro-17-phenyl-18,19,20-trinor PGF_{2α} ethyl amide; 13,14-dihydro-Fluprostenol isopropyl ester; 13,14-dihydro-fluprostenol 1,15-lactone; 13,14-dihydro-17-phenyl-18,19,20-trinor PGF₂α 1,15-lactone; 13,14-dihydro-17,17,18,11-tetradehydro -PGF₂α 1,13-lactone; 13,14-dihydro-16-phenoxy-17,18,19,20-tetranor PGP₂α 1,15-lactone; cloprostenol N-ethyl amide. Cayman Chemical, for example, provides the preferred 1,15-lactone forms of PGF_{2α} analogs.

It is, of course, contemplated that a formulation in accordance with the present invention will could include combinations of more than one prostaglandin and/or antimicrobial peptide.

A topical ophthalmic composition or formulation, in accordance with the present invention, broadly comprises a pharmaceutically acceptable carrier and a therapeutically effective amount of at least one compound having a chemical structure in accordance with Formulae I and/or II in combination with an antimicrobial peptide preservative.

In typical formulations, the carrier for the active agent(s) would be an aqueous solution buffered to physiologically-acceptable pH levels with an acidic or basic pH adjusting agent. Of course, the osmolarity of the solution would be adjusted with osmolarity adjusting agents, such as sodium chloride (NaCl) or potassium chloride (KCl), as is known in the art. Other ingredients, excipients, and additives which may be desirable to use in the ophthalmic preparations of the present invention include antioxidants, co-solvents and viscosity building agents.

In forming compositions for topical administration, the prostaglandin compounds of the present invention are generally formulated as between about 0.0001 to about 0.8 percent by weight (wt %) solutions in an aqueous solution buffered to a pH between 4.5 to 8.0 with a suitable buffering agent, and most preferably between 7.0-7.6. The compounds are preferably formulated as between about 0.0003 to about 0.3 wt % and, most preferably, between about 0.003 and about 0.03 wt %.

Ophthalmic products are typically packaged in multidose form, which generally require the addition of preservatives to prevent microbial contamination during use. In accordance with the invention, the formulation preservative is a naturally-occurring or recombinant antimicrobial peptide, including the antimicrobial peptides listed hereinabove in Table 1, lactoferrin, and lactoferricin. The antimicrobial peptide typically would be employed at a concentration between about 0.001% and 1.0% by weight.

Prostaglandins, and particularly ester or amide derivatives of prostaglandins, typically have limited solubility in water and therefore may require a surfactant or other appropriate co-solvent in the composition. Such co-solvents include: Polysorbate 20, 60 and 80; Pluronic F-68, F-84 and P-103; Tyloxapol^{®}; Cremophor^{®} EL; sodium dodecyl sulfate; glycerol; PEG 400; propylene glycol; cyclodextrins; or other agents known to those skilled in the art. Such co-solvents are typically employed at a concentration between about 0.01% and about 2% by weight.

Viscosity greater than that of simple aqueous solutions may be desirable to increase ocular absorption of the active compound, to decrease variability in dispensing the formulations, to decrease physical separation of components of a suspension or emulsion of formulation and/or otherwise to improve the ophthalmic formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose or other agents known to those skilled in the art. Such agents are typically employed at a concentration between about 0.01% and about 2% by weight.

The compounds of Formulae I and II are active in lowering the intraocular pressure in humans and other animals and are therefore useful in the treatment of glaucoma and other disorders that cause an increase in intraocular pressure. Therefore, in a method of use aspect of the invention, a therapeutically effective amount of at least one compound having the structure of Formulae I and II are administered to an affected eye to treat increased intraocular pressure in an ophthalmic formulation containing an antimicrobial peptide preservative.

The preferred route of administration is via the topical application of sterile ophthalmic solutions directly into the eye. The dosage range for topical administration is between about 0.1 and 100 micrograms (µg) per eye per day, and is most preferably between 1 and 10 µg per eye per day. While the precise regimen is left to the discretion of the clinician, it is recommended that the resulting solution be topically applied by placing one drop in each eye one or two times a day.

### DETAILED DESCRIPTION OF THE INVENTION

The following Examples are representative of the type of pharmaceutical preparations or formulations in accordance with the principles of the invention and that are contemplated for topical application in the treatment of glaucoma.

### EXAMPLE 1

| **Ingredient** | **Amount (wt%)** |
|---|---|
| Fluprostenol 1,15 Lactone | 0.002 |
| Dextran 70 | 0.10 |
| Hydroxypropyl Methylcellulose | 0.30 |
| Sodium Chloride | 0.77 |
| Potassium Chloride | 0.12 |
| Lactoferrin (human recombinant) | 0.10 |
| HCl and/or NaOH | pH = 7.0-7.6 |
| Purified Water | q.s. to 100% |

### EXAMPLE 2

| **Ingredient** | **Amount (wt%)** |
|---|---|
| 13,14-dihydro-17-phenyl-18,19,20-trinor PGF | |
| isopropyl ester | 0.004 |
| Dextran 70 | 0.10 |
| Hydroxypropyl Methylcellulose | 0.30 |
| Sodium Chloride | 0.77 |
| Potassium Chloride | 0.12 |
| Lactoferrin (human recombinant) | 0.10 |
| HCl and/or NaOH | pH = 7.0-7.6 |
| Purified Water | q.s. to 100% |

### EXAMPLE 3

| **Ingredient** | **Amount (wt%)** |
|---|---|
| Cloprostenol N-ethyl amide | 0.02 |
| Dextran 70 | 0.10 |
| Hydroxypropyl Methylcellulose | 0.30 |
| Sodium Chloride | 0.77 |
| Potassium Chloride | 0.12 |
| Lactoferrin (human recombinant) | 0.10 |
| HC! and/or NaOH | pH = 7.0-7.6 |
| Purified Water | q.s. to 100% |

### EXAMPLE 4

| **Ingredient** | **Amount (wt%)** |
|---|---|
| 13,14-dihydro-fluprostenol 1,5-lactone | 0.003 |
| Dextran 70 | 0.10 |
| Hydroxypropyl Methylcellulose | 0.30 |
| Sodium Chloride | 0.77 |
| Potassium Chloride | 0.12 |
| Pexiganan | 0.10 |
| HCl and/or NaOH | pH = 7.0-7.6 |
| Purified Water | q.s. to 100% |

### EXAMPLE 5

| **Ingredient** | **Amount (wt%)** |
|---|---|
| 13,14-dihydro-17-phenyl-18,19,20-trinor PGF_{2α} | |
| 1,15-lactone | 0.004 |
| Dextran 70 | 0.10 |
| Hydroxypropyl Methylcellulose | 0.30 |
| Sodium Chloride | 0.77 |
| Potassium Chloride | 0.12 |
| Pexiganan | 0.10 |
| HCl and/or NaOH | pH = 7.0-7.6 |
| Purified Water | q.s. to 100% |

### EXAMPLE 6

| **Ingredient** | **Amount (wt%)** |
|---|---|
| 13,14-dihydro-17-phenyl-18,19,20-trinor PGF_{2α} | |
| 1,15-lactone | 0.004 |
| Dextran 70 | 0.10 |
| Hydroxypropyl Methylcellulose | 0.30 |
| Sodium Chloride | 0.77 |
| Potassium Chloride | 0.12 |
| Buforin II | 0.10 |
| HCl and/or NaOH | pH = 7.0-7.6 |
| Purified Water | q.s. to 100% |

### EXAMPLE 7

| **Ingredient** | **Amount (wt%)** |
|---|---|
| 13,14-dihydro-17-phenyl-18,19,20-trinor PGF_{2α} | |
| ethyl amide | 0.001 |
| Dextran 70 | 0.10 |
| Hydroxypropyl Methylcellulose | 0.30 |
| Sodium Chloride | 0.77 |
| Potassium Chloride | 0.12 |
| Buforin II | 0.10 |
| HCl and/or NaOH | pH = 7.0-7.6 |
| Purified Water | q.s. to 100% |

### EXAMPLE 8

| **Ingredient** | **Amount (wt%)** |
|---|---|
| 13,14-dihydro-17-phenyl-18,19,20-trinor PGF_{2α} | |
| isopropyl ester | 0.004 |
| Dextran 70 | 0.10 |
| Hydroxypropyl Methylcellulose | 0.30. |
| Sodium Chloride | 0.77 |
| Potassium Chloride | 0.12 |
| Buforin II | 0.10 |
| HCl and/or NaOH | pH - 7.0-7.6 |
| Purified Water | q.s. to 100% |

### EXAMPLE 9

| **Ingredient** | **Amount (wt%)** |
|---|---|
| Fluprostenol 1,15-lactone | 0.004 |
| Dextran 70 | 0.10 |
| Hydroxypropyl Methylcellulose | 0.30 |
| Sodium Chloride | 0.77 |
| Potassium Chloride | 0.12 |
| Bactenecin 1 | 0.10 |
| HCl and/or NaOH | pH= 7.0-7.6 |
| Purified Water | q.s. to 100% |

## Claims

1. An ophthalmic formulation comprising a prostaglandin of the F-Series and an antimicrobial peptide.

2. The ophthalmic formulation of claim 1 wherein the prostaglandin is a prodrug selected from the group consisting of prostaglandin esters, prostaglandin , amides, and prostaglandin lactones.

3. The ophthalmic formulation of claims 1 and 2 wherein the prostaglandin is an analog of PGF_{2α}.

4. The ophthalmic formulation of claim 3 wherein the prostaglandin is a macrocyclic internal 1,15-lactone of a PGF_{2α} analog.

5. The ophthalmic formulation of claim 4 wherein the prostaglandin is an internal 1,15-lactone of a PGF_{2α} analog.

6. An ophthalmic formulation of a prostaglandin having the general Formula I in combination with an antimicrobial peptide: 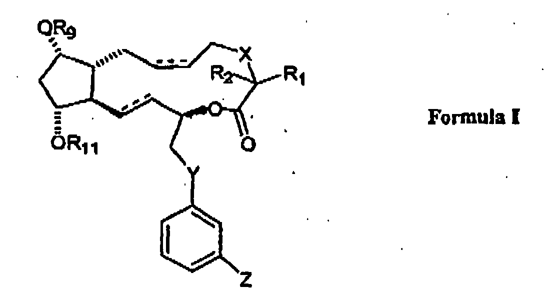 wherein X is O, S, NH or CH_{2;}
R₁ and R₂ are the same and are either H, CH₃ or F;
R₉ is H, or C₁-C₂₀ straight chain, saturated or unsaturated or branched acyl;
R₁₁ is H, or C₁-C₂₀ straight chain, saturated or unsaturated or branched acyl;
---- represents any combination of a single bond, or a *cis* or *trans* double bond;
Z is H, Cl, Br, I, CF₃, CH₃, or C₁-C₁₀ straight chain or branched alkyl;
Y is O, S, NH or CH₂.

7. The ophthalmic formulation of claims 1 and 6 wherein the antimicrobial peptide is of human origin.

8. The ophthalmic formulation of claims 1 and 6 wherein the antimicrobial peptide is lactoferrin.

9. An ophthalmic formulation of a prostaglandin having the general Formula II, as a free acid or prodrug, in combination with an antimicrobial peptide: 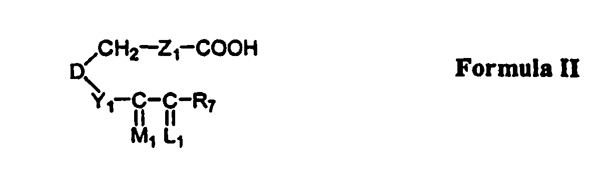 wherein D is 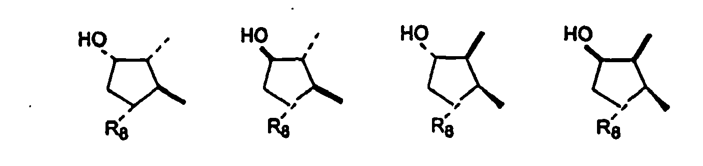 wherein R₈ is hydrogen or hydroxy;
wherein L₁ is 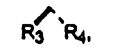 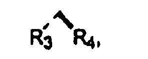 or a mixture of 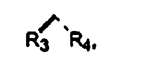 and 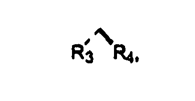 wherein R₃ and R₄ are hydrogen, methyl or fluoro, being the same or different, with the proviso that one of R₃ and R₄ is fluoro, only when the other is hydrogen or fluoro;
wherein M₁ is 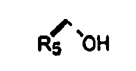 or 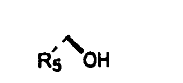 wherein R₅ is hydrogen or methyl;
wherein R₇ is -(CH₂)*ₘ* -CH₃, wherein *m* is one to 5,
inclusive, *cis* -CH=CH-CH₂-CH₃, or 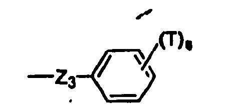 wherein T is chloro, fluoro, trifluoromethyl, alkyl of one to 3 carbon atoms, inclusive, or alkoxy of one to 3 carbon atoms, inclusive, the various T's being the same or different, s is zero, one, 2, or 3, and Z₃ is oxa or methylene, with the proviso, that not more than two T's are other than alkyl, and the further proviso that Z₃ is oxa only when R₃ and R₄ are hydrogen or methyl, being the same or different;
wherein Y₁ is trans -CH=CH-, -CH₂CH₂-,
cis-CH=CH-, or -C≡C-; and
wherein Z₁ is
(1) cis-CH=CH-CH₂-(CH₂)*_{g}*-CH₂-,
(20) cis-CH=CH-CH₂-(CH₂)*_{g}*-CF₂-,
(3) cis-CH₂-CH=CH-(CH₂)*_{g-}*CH₂-,
(4) -(CH₂)₃-(CH₂)_{*g*-}CH₂₋,
(5) -(CH₂)₃-(CH₂)*_{g}*-CF₂-,
(6) -CH₂-O-CH₂-(CH₂)*_{g}*-CH₂-, 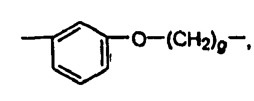 and 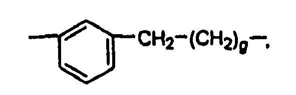 wherein *g* is one, 2, or 3.

## Patentansprüche

1. Ophthalmische Formulierung, welche ein Prostaglandin der F-Serie und ein antimikrobielles Peptid aufweist.

2. Ophthalmische Formulierung nach Anspruch 1, wobei es sich bei dem Prostaglandin um ein Propharmakon handelt, das aus der Gruppe ausgewählt ist, die aus Prostaglandinestern, Prostaglandinamiden und Prostaglandinlaktonen besteht.

3. Ophthalmische Formulierung nach Anspruch 1 und 2, wobei es sich bei dem Prostaglandin um ein Analog von PGF_{2α} handelt.

4. Ophthalmische Formulierung nach Anspruch 3, wobei es sich bei dem Prostaglandin um ein makrozyklisches internes 1,15-Lakton eines PGF_{2α}-Analogs handelt.

5. Ophthalmische Formulierung nach Anspruch 4, wobei es sich bei dem Prostaglandin um ein internes 1,15-Lakton eines PGF_{2α}-Analogs handelt.

6. Ophthalmische Formulierung eines Prostaglandins, das die allgemeine Formel I aufweist, in Kombination mit einem antimikrobiellen Peptid: 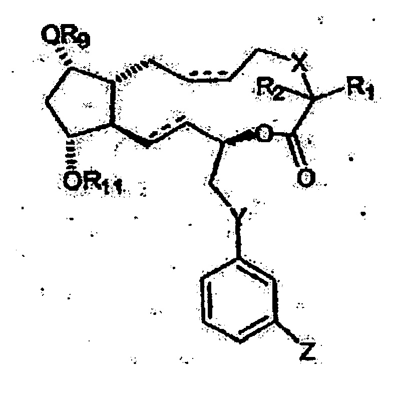 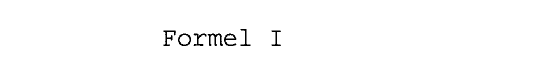 wobei X O, S, NH oder CH₂ ist;
R₁ und R₂ gleich und entweder H, CH₃ oder F sind;
R₉ H oder ein geradkettiges, gesättigtes oder ungesättigtes oder verzweigtes C₁-C₂₀-Acyl ist;
R₁₁ H oder ein geradkettiges, gesättigtes oder ungesättigtes oder verzweigtes C₁-C₂₀-Acyl ist;
---- eine beliebige Kombination einer einfachen Bindung oder einer cis- oder trans-Doppelbindung darstellt;
Z H, Cl, Br, I, CF₃, CH₃ oder ein geradkettiges oder verzweigtes C₁-C₂₀-Acyl ist;
Y O, S, NH oder CH₂ ist.

7. Ophthalmische Formulierung nach Anspruch 1 und 6, wobei das antimikrobielle Peptid menschlichen Ursprungs ist.

8. Ophthalmische Formulierung nach Anspruch 1 und 6, wobei es sich bei dem antimikrobiellen Peptid um Laktoferrin handelt.

9. Ophthalmische Formulierung eines Prostaglandins, das die allgemeine Formel II aufweist, als freie Säure oder Propharmakon in Kombination mit einem antimikrobiellen Peptid: 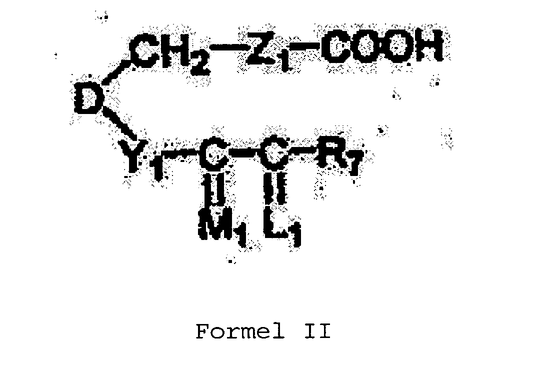 wobei D 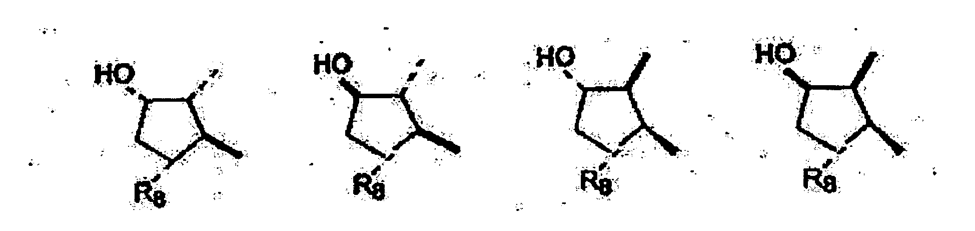 ist, wobei R₈ Wasserstoff oder Hydroxy ist;
wobei L₁ 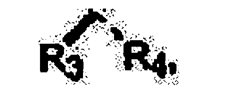 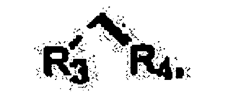 oder ein Gemisch aus 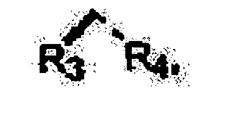 und 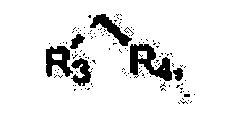 ist, wobei R₃ und R₄ Wasserstoff, Methyl oder Fluor sind und entweder gleich oder unterschiedlich sind, vorausgesetzt, dass eines von R₃ oder R₄ Fluor ist, sofern das Andere Wasserstoff oder Fluor ist;
wobei M₁ 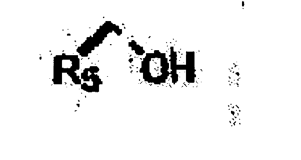 oder 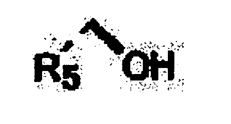 ist, wobei R₅ Wasserstoff oder Methyl ist;
wobei R₇-(CH₂)*ₘ*-CH₃ ist, wobei *m* einschließlich eins bis 5, cis-CH=CH-CH₂-CH₃ oder 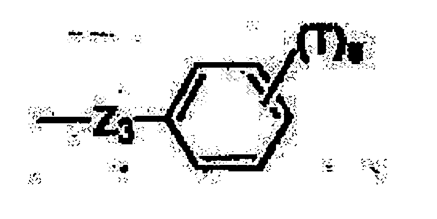 ist, wobei T Chlor, Fluor, Trifluormethyl, Alkyl aus einschließlich einem bis 3 Kohlenstoffatomen oder Alkoxy mit einschließlich einem bis 3 Kohlenstoffatomen ist, wobei die verschiedenen T-Reste gleich oder unterschiedlich sind, s null, eins, 2 oder 3 ist und Z₃ Oxa oder Methylen ist, vorausgesetzt, dass es sich bei nicht mehr als zwei T-Resten nicht um Alkyl handelt, und des Weiteren vorausgesetzt ist, dass Z₃ nur dann Oxa ist, wenn R₃ und R₄ Wasserstoff oder Methyl und gleich oder unterschiedlich sind;
wobei Y₁ trans-CH=CH-, -CH₂CH₂-,
cis-CH=CH- oder -C 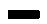 C- ist und
wobei Z₁
(1) cis-CH=CH-CH₂-(CH₂)*_{g}*-CH₂-,
(2) cis-CH=CH-CH₂-(CH₂)*_{g}*-CF₂-,
(3) cis-CH₂-CH=CH-(CH₂)*_{g}*-CH₂-,
(4) -(CH₂)₃-(CH₂)*_{g}*-CH₂-,
(5) -(CH₂)₃-(CH₂)*_{g}*-CF₂-,
(6) -CH₂-O-CH₂-(CH₂)*_{g}*-CH₂-, 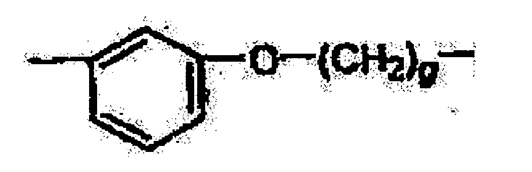 und 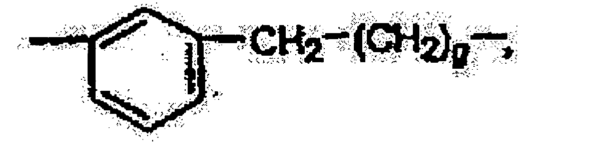
ist, wobei *g* eins, 2 oder 3 ist.

## Revendications

1. Formulation ophtalmique comprenant une prostaglandine de la série F et un peptide antimicrobien.

2. Formulation ophtalmique selon la revendication 1, dans laquelle la prostaglandine est un précurseur choisi dans le groupe constitué d'esters de prostaglandine, d'amides de prostaglandine et de lactones de prostaglandine.

3. Formulation ophtalmique selon les revendications 1 et 2, dans laquelle la prostaglandine est un analogue de la PGF_{2α}.

4. Formulation ophtalmique selon la revendication 3, dans laquelle la prostaglandine est une 1,15-lactone interne macrocyclique d'un analogue de la PGF_{2α}.

5. Formulation ophtalmique selon la revendication 4, dans laquelle la prostaglandine est une 1,15-lactone interne d'un analogue de la PGF_{2α}.

6. Formulation ophtalmique d'une prostaglandine ayant la formule générale 1 en combinaison avec un peptide antimicrobien : 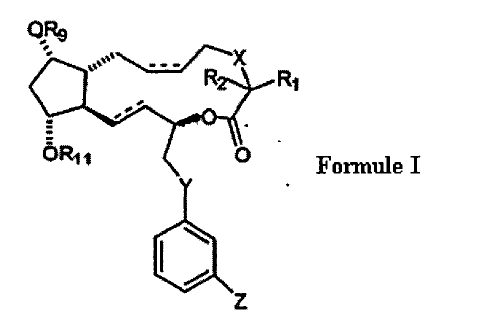 dans laquelle X représente O, S, NH ou CH₂ ;
R₁ et R₂ sont identiques et représentent soit H soit CH₃ soit F ;
R₉ représente H ou un groupe acyle en C₁ à C₂₀ à chaîne linéaire, saturée ou insaturée ou ramifiée ;
R₁₁ représente H ou un groupe acyle en C₁ à C₂₀ à chaîne linéaire, saturée ou insaturée ou ramifiée ;
---- représente toute combinaison d'une liaison simple ou d'une double liaison en *cis* ou *trans ;*
Z représente H, Cl, Br, I, CF₃, CH₃ ou un groupe alkyle en C₁ à C₁₀ à chaîne linéaire ou ramifiée ;
Y représente O, S, NH ou CH₂.

7. Formulation ophtalmique selon les revendications 1 et 6, dans laquelle le peptide antimicrobien est d'origine humaine.

8. Formulation ophtalmique selon les revendications 1 et 6, dans laquelle le peptide antimicrobien est la lactoferrin.

9. Formulation ophtalmique d'une prostaglandine ayant la formule générale II, sous la forme d'un acide libre ou d'un précurseur, en combinaison avec un peptide antimicrobien : 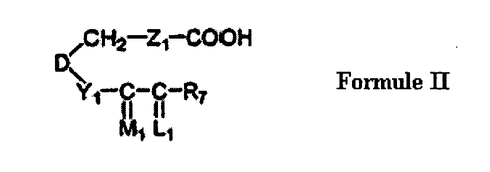 dans laquelle D représente 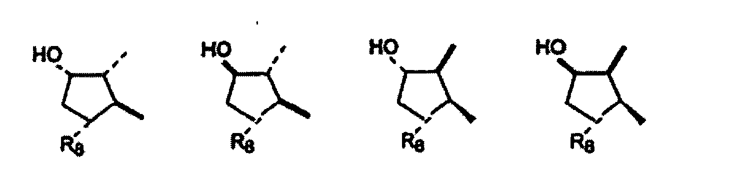 où R₈ représente un atome d'hydrogène ou un groupe hydroxy ;
dans laquelle L₁ représente 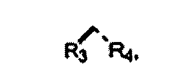 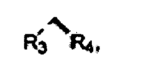 ou un mélange de 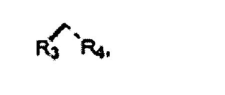 et 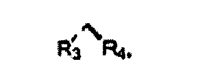 où R₃ et R₄ représentent un atome d'hydrogène, un groupe méthyle ou fluoro, étant identiques ou différents, à condition que l'un de R₃ et R₄ représente un groupe fluoro, seulement lorsque l'autre représente un atome d'hydrogène ou un groupe fluoro ; dans laquelle M₁ représente 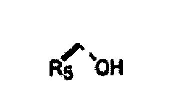 ou 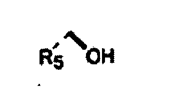 où R₅ représente un atome d'hydrogène ou un groupe méthyle ;
dans laquelle R₇ représente -(CH₂)*ₘ*-(CH₃), où *m* vaut un à 5, compris, *cis*-CH=CH-CH₂-CH₃, ou 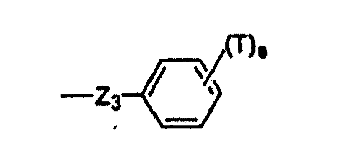 où T représente un groupe chloro, fluoro, trifluorométhyle, alkyle d'un à 3 atomes de carbone, compris, ou alcoxy d'un à 3 atomes de carbone, compris, les divers T étant identiques ou différents, *s* vaut zéro, un, 2 ou 3, et Z₃ représente un groupe oxa ou méthylène, à condition que pas plus de deux T soient différents d'un groupe alkyle, et à condition en outre que Z₃ représente un groupe oxa seulement lorsque R₃ et R₄, identiques ou différents, représentent un atome d'hydrogène ou un groupe méthyle; dans laquelle Y₁ représente *trans*-CH=CH-, -CH₂CH₂-, *cis*-CH=CH- ou -C≡C- ; et
dans laquelle Z₁ représente
(1) *cis*-CH=CH-CH₂-(CH₂)*_{g}*-CH₂-,
(20) cis-CH=CH-CH₂-(CH₂)*_{g}* CF₂-,
(3) cis-CH₂-CH=CH-(CH₂)*_{g}*-CH₂-,
(4) -(CH₂)₃-(CH₂)*_{g}*-CH₂-,
(5) -(CH₂)₃-(CH₂)*_{g}*-CF₂-,
(6) -CH₂-O-CH₂-(CH₂)*_{g}*-CH₂-, 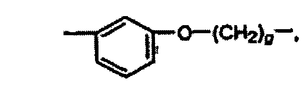 et 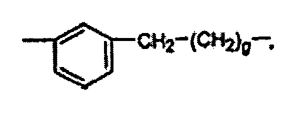
où *g* vaut un, 2 ou 3.
